# EUROPEAN PATENT SPECIFICATION

(11) **EP 3 038 812 B1**
(45) Date of publication and mention of the grant of the patent: **20.11.2019**
(21) Application number: 14838968.7
(22) Date of filing: 22.08.2014
(51) Int. Cl.: B29C 65/70, B29C 45/14, A61M 39/12, F16L 33/34, A61M 39/10, B29C 65/00, F16L 47/32, B29K 83/00, B29L 31/24, B29K 105/24

(54) **METHOD AND DEVICE FOR OVERMOLDING UV-CURABLE MATERIAL OVER POLYMER INSERTS**
VERFAHREN UND VORRICHTUNG ZUM ÜBERSPRITZEN EINES UV-HÄRTBAREN MATERIALS ÜBER POLYMEREINSÄTZE
PROCÉDÉ ET DISPOSITIF POUR SURMOULER UN MATÉRIAU DURCISSABLE PAR UV SUR DES INSERTS EN POLYMÈRE

(30) Priority: 29.08.2013 US 201361871752 P
(43) Date of publication of application: 06.07.2016
(73) Proprietor: Alphinity, LLC, Carson City, NV 89706 (US)
(72) Inventor: GAGNE, Michael C., Reno, Nevada 89511 (US); CATES, Steven V., Lakewood, CA 90713 (US); RICHARDS, Dean, Simi Valley, California 93063 (US)
(74) Representative: Lecomte & Partners
(86) International application number: PCT/US2014/052383
(87) International publication number: WO 2015/031207

(56) References cited:
- WO-A1-2012/170008
- WO-A2-2007/048072
- US-A- 4 668 217
- US-A- 5 411 300
- US-A1- 2008 267 699
- US-A1- 2012 223 517
- US-A1- 2013 043 676
- Rado Gummi Gmbh ET AL: "Fachmagazin für die Polymerindustrie UV-vernetzendes Silicon Vulkametrie Lauffl ächenmischungen Spritzgießsimulation Heizzeitreduzierung UV-vernetzendes Silicon", , 1 February 2013 (2013-02-01), XP055322583, Retrieved from the Internet: URL:http://www.momentivespecialtychemicals .com/uploadedFiles/Product_Literature/Sili cones/Elastomers/GAK.pdf [retrieved on 2016-11-24]

## Description

### Field of the Invention

The field of the invention generally relates to fluid management devices and, in particular, connectors or interfaces used therein. More specifically, the invention pertains to connectors or interfaces used by pharmaceutical and biological applications or other hygienic process industries involving silicone tubing or other conduits.

### Background of the Invention

Many commercial products are produced using chemical as well as biological processes. Pharmaceuticals, for example, are produced in commercial quantities using scaled-up reactors and other equipment. So-called biologics are drugs or other compounds that are produced or isolated from living entities such as cells or tissue. Biologics can be composed of proteins, nucleic acids, or complex combinations of these substances. They may even include living entities such as cells. In order to produce biologics on a commercial scale, sophisticated and expensive equipment is needed. In both pharmaceutical and biologics, for example, various processes need to occur before the final product is obtained. For example, in the case of biologics, cells may be grown in a growth chamber or the like and nutrients may need to be carefully modulated into the growth chamber. Waste products produced by cells may also have to be removed on a controlled basis from the fermentation chamber. As another example, biologic products produced by living cells or other organisms may need to be extracted and concentrated. This process may involve a variety of filtration and separation techniques.

Because there are a number of individual processes required to be produce the final product, various reactants, solutions, and washes are often pumped or otherwise transported to various subsystems using conduits and associated valves. These systems may be quite cumbersome and organizationally complex due to the large numbers of conduits, valves, sensors, and the like that may be needed in such systems. Not only are these systems visually complex (e.g., resembling spaghetti) they also include many components that are required to sterilized between uses to avoid cross-contamination issues. Indeed, the case of drug and biologic preparation, the Federal Food and Drug Administration (FDA) is becoming increasingly strict on cleaning, sterilization or bio-burden reduction procedures that are required for drug and pharmaceutical preparations. This is particularly of a concern because many of these products are produced in batches which would require repeated cleaning, sterilization or bio-burden reduction activities on a variety of components.

Some attempts have been made at incorporating various disposable elements into the system. For example, conduits or lines connecting various systems or elements have been made of silicone. Unfortunately, silicone tubing or conduits often have to be reinforced along their periphery to avoid the possibility of leakage through an aneurysm or the like that develops at the wall of the tubing. Reinforced silicone tubing is, however, rather expensive and is not as flexible as un-reinforced silicone.

In addition, silicone tubing often connects to other connectors or junctions (e.g., Tees, Wyes, unions, elbows) as part of the overall system. Current interfaces between silicone tubing and various connectors are deficient in a number of respects. First, current interfaces are limited in the pressure that they can handle. For example, existing connections between silicone tubing and connectors can operate at pressures at or below about 2.07 bar (30 psi). There is a growing need to utilize higher pressures within systems because higher pressures (as well as larger tubing diameters) enables higher throughput. In addition, the interface between the silicone tubing and the connector often requires an external clamping member such as a zip tie which surrounds the junction between the silicone tubing and the inner connector. Unfortunately, the presence of these zip ties or other clamping members on the exterior tubing can lead to tears or breakages in the disposable gloves used by workers which jeopardizes the sterile environment in which these systems are typically used.

Another disadvantage of current junctions and interfaces between silicone tubing and various devices is the fact that they introduce another material that contacts the pharmaceutical or biological component that is carried through the system. While plastics and silicone have known interactions with pharmaceutical and biological components, other materials have unknown or deleterious effects on the final product. This tertiary contact with the product may jeopardize the purity and sterility of the ultimate product. For example, connectors and junctions are sometimes created using an internal mandrel. During the manufacturing process, the use of the mandrel produces flash which may have to be manually removed prior to final assembly and use. Inevitably, however, some flash may remain which can clog or otherwise disrupt the process. It also introduces a variable in the form of the overmolding or joining silicone that is now a product contact material that needs to be monitored and qualified for the manufacturer's validation documentation.

U.S. Patent No. 5,447,341 illustrates a molded hose branch of rubber that is formed by slipping completely vulcanized rubber hose lengths with ends onto the free ends of a plastic tubing branching piece. The assembly is placed into a mold and a thermoplastic material such as a blended mixture of polyproplylene and EPDM is injected molded around the assembly so as to join the thermoplastic material to the rubber hose lengths. The problem with such a construction in connection with systems that rely on silicone, in conventional silicone molding processes, the temperature at which silicone cures would cause the inner plastic branching piece to melt. Standard platinum cured silicone may require extended temperature of around 180 °C to cross-link silicone. These high temperatures would melt or otherwise destroy the underlying plastic branching piece.

U.S. Patent No. 6,290,265 discloses a tubing and connector assembly that includes a multi-lumen connector having at least three flexible tubes molded onto the connector. The process of making the connector and tubing assembly includes forming a first part of the connector with two tubes molded therein and then removing an internal mold member prior to molding the final connector portion and third tube in place. As noted above, in this method, an internal mandrel-type structure is used as the method uses an inner mold member and mold insert.

U.S. Patent No. 6,432,345 illustrates a single-step method of manufacturing a silicone tube manifold. The method forms, in a single step, a silicone manifold interconnecting a plurality of silicone tubes. The method is performed by providing a silicone insert piece having a network of interior channels dimensioned the same as the silicone tube interiors. Solid non-silicone plugs are inserted into the insert piece and the ends of the silicone tubes and the resulting assembly is placed in a mold cavity. Liquid silicone is added to the mold cavity and is then heated and cured to form the desired manifold interconnecting the tubes. The manifold is then removed from the mold and a source of pressurized air is used to blow out the plugs.

U.S. Patent No. 8,424,923 illustrates a fluid transfer assembly for transporting medicinal substances. The fluid transfer assembly includes flexible tubes and a manifold each comprising silicone. The manifold has an inner protrusion and connector portions having inner walls. Free ends of the tubes are inserted into complementary configured inner walls of the connector portions until each of the free ends abut the inner protrusion, which creates a continuous uninterrupted passageway between the inner bores of the tubes through the manifold. This patent discloses the formation of an outer capsule by using silicone rubber that is introduced into a molding assembly.

WO 2007/048072 shows a fluid transfer assembly comprising a manifold, flexible tubes and an outer capsule made from silicone based materials.

### Summary of the Invention

The invention as defined in claim 1 and 9 aims at overcoming the drawbacks of the prior art. The subject-matter of claim 1 defines a fluid management assembly according to the invention. The subject-matter of claim 9 defines a method of forming a fluid management assembly according to the invention.

### Brief Description of the Drawings

FIG. 1 illustrates a fluid management assembly according to one embodiment.
FIG. 2A illustrates a fluid management assembly with a connector formed as a wye.
FIG. 2B illustrates a fluid management assembly with a connector formed as a connector.
FIG. 2C illustrates a fluid management assembly with a connector formed as an elbow.
FIG. 2D illustrates another embodiment of a connector in the form of a manifold.
FIG. 2E illustrates another embodiment of a connector in the form of a reducer.
FIG. 3 illustrates a mold containing a connector and tubing segments ready to receive the liquid polymer.
FIG. 4 illustrates a fluid management assembly according to another embodiment.

### Detailed Description of the Illustrated Embodiments

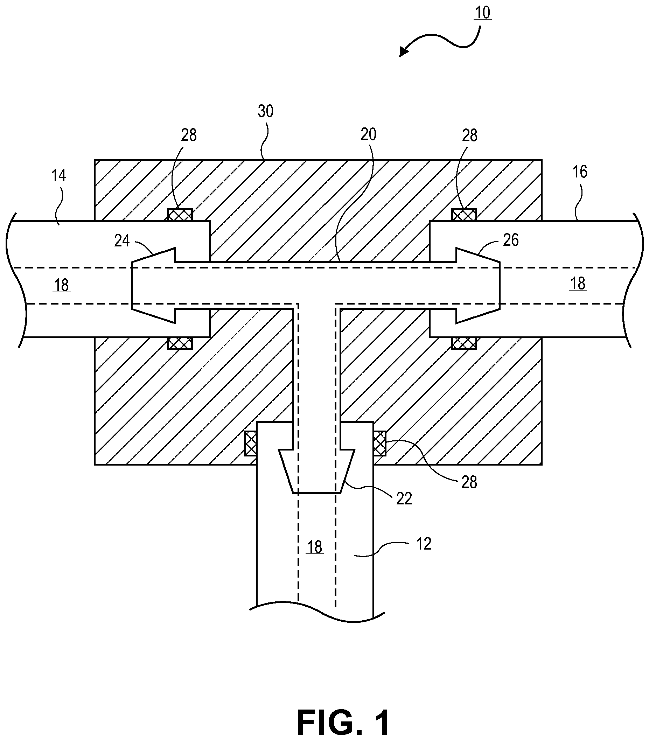

FIG. 1 illustrates a fluid management assembly 10 according to one embodiment. The fluid management assembly as illustrated in FIG. 1 includes a first tubing segment 12, a second tubing segment 14, and a third tubing segment 16. Each tubing segment 12, 14, 16 includes a lumen or passageway therein 18 for the passage of fluids. The tubing segments 12, 14, 16 may be formed from a number of materials including silicone, braided and/or reinforced silicone, a thermoplastic elastomer (TPE) or a thermoplastic rubber (TPR). The tubing segments 12, 14, 16 can have any number of dimensions but the invention is particularly suited for larger diameter tubing (e.g., between ¾ inch ID and 2 inch ID although this range is not limiting). The free ends of the tubing segments 12, 14, 16 are connected to ends of a connector 20. The connector 20 illustrated in FIG. 1 includes ends 22, 24, and 26. The ends 22, 24, 26 are preferably barbed ends as illustrated although in other embodiments the ends 22, 24, 26 may not be barbed. A frictional interface is formed where each tubing segment 12, 14, 16 joins with the respective ends 22, 24, and 26 of the connector 20.

In the embodiment of FIG. 1, one tubing segment (e.g., segment 12) may serve as an inlet while the remaining tubing segments 14, 16 may serve as outlets for fluid. As described herein, a variety of different configurations of the connector 20 are contemplated. In all the embodiments, there is at least one inlet and at least one outlet on the connector 20. Still referring to FIG. 1, clamps 28 are illustrated surrounding the tubing segments 12, 14, 16 at interface formed between the tubing segments 12, 14, 16 and the ends 22, 24, 26. The clamps 28 are optional and may increase the robustness of the fluid management assembly 10 enabling the same to operate at higher pressures. The clamps 28 may take any number of forms. For example, the clamps 28 may include hose clamps or zip ties commonly used in fluid applications.

The connector 20 may be made from a number of polymer materials. For example, the connector 20 may be made from a plastic material. Plastic materials include standard thermoplastics and polyolefins such as polyethylene (PE) and polypropylene (PP). The connector 20 may also be formed from fluoropolymers such as polyvinylidene fluoride (PVDF) or Perfluoroalkoxy (PFA), Polytetrafluoroethylene (PTFE), Polycarbonate (which may be more thermally resistant) and the like. Typically, the connector 20 is made from a polymer material that would be compromised upon exposure to high thermal temperatures like those experienced in conventional thermal curing processes.

Still referring to FIG. 1, the fluid management assembly 10 includes a polymer-based overmolding 30. The polymer-based overmolding 30 as explained in more detail below is a solid section that becomes solid after exposure to ultraviolet (UV) radiation during a curing process. The overmolding 30 covers the entirety of the connector 20 as well as the interface formed between tubing segments 12, 14, 16 and ends 22, 24, and 26. In this regard, the overmolding 30 adds additional strength to the respective interfaces formed between the tubing segments 12, 14, 16 and the ends 22, 24, and 26. This enables the fluid management assembly 10 to be very robust and can operate at very high operating pressures. For example, typical connectors are believed to operate at maximum pressures of around 1.72 - 2.07 bar (25-30 psi). However, in the fluid management assembly 10 is able to operate at much higher pressures - e.g., above 3.45 bar 50 (psi) or6.89 bar (100 psi). The presence of the optional clamps 28 can be used for applications requiring particularly high pressures.

In one aspect of the invention, the polymer-based overmolding 30 is silicone based. For example, SILOPREN available from Momentive Performance Materials Inc. (Albany, NY) is a UV cured Liquid Silicone Rubber (LSR) that may be used as the overmolding 30. SILOPREN is a two-component LSR what uses a mixing ratio of 100:2. Another example of a UV cured silicone is ADDISIL available from Momentive Performance Materials Inc. (Albany, NY) which offers high cure speed at room temperatures. ADDISIL silicone rubber is a two component solution that uses a mixing ratio of 100:0.5 (Rubber Base:Catalyst). The benefits of using a UV curable overmolding 30 are numerous. First, there is no heating of the materials as is required in conventional silicone curing techniques. Thus, the overmolding 30 may be formed at substantially room temperatures. If the polymer based connector 20 described herein were to be overmolded with conventional thermally-cured silicone, the connector 20 itself would melt, deform, or otherwise fail during the curing process. Here, using the UV curing process, the connector 20 remains unaffected by the curing process. Because no heating is required, the process also has low energy consumption. Further, the UV curing process uses less equipment and can increase throughput as heating up and cool-down tend to consume a considerable amount of time. Another example of a UV curable silicone is the SEMICOSIL UV product made by Wacker Chemie AG (Munich, Germany).

Standard liquid silicone rubbers are processed in molds generally at temperatures between 180 and 200°C. UV-cured LSR parts can be made with transparent molds cured by UV light as explained herein. UV curing typically occurs at ambient or slightly above ambient temperatures (e.g., 25-40°C). For thick parts, UV curing is advantageous because the product may cross-link much more quickly than with thermal curing because the low thermal conductivity of the silicone rubber is not a factor. Another advantage of UV curing is that the curing is initiated when the light is first turned on rather than on first contact with the heated mold. This difference eliminates the problem of silicone rubber scorching. Related to this, the liquid silicone (or other polymer) can be pumped into the mold relatively slowly and at low pressures.

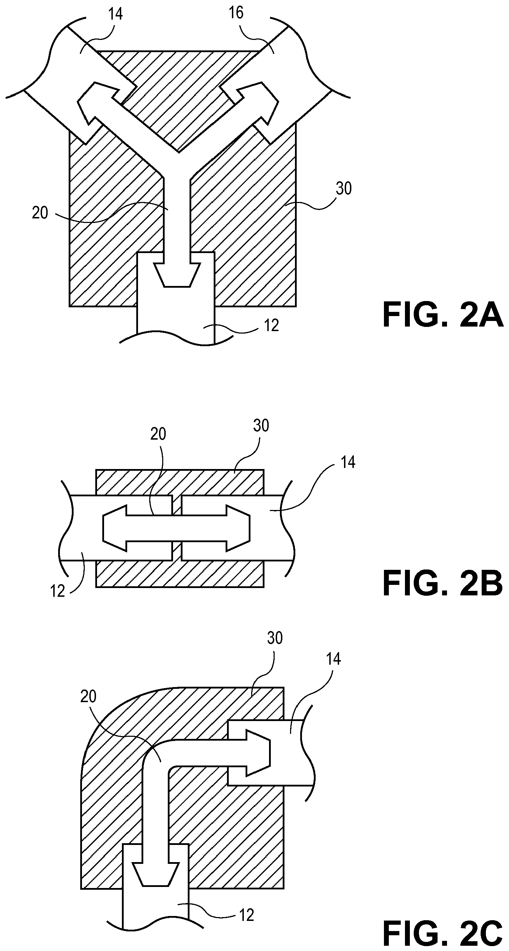

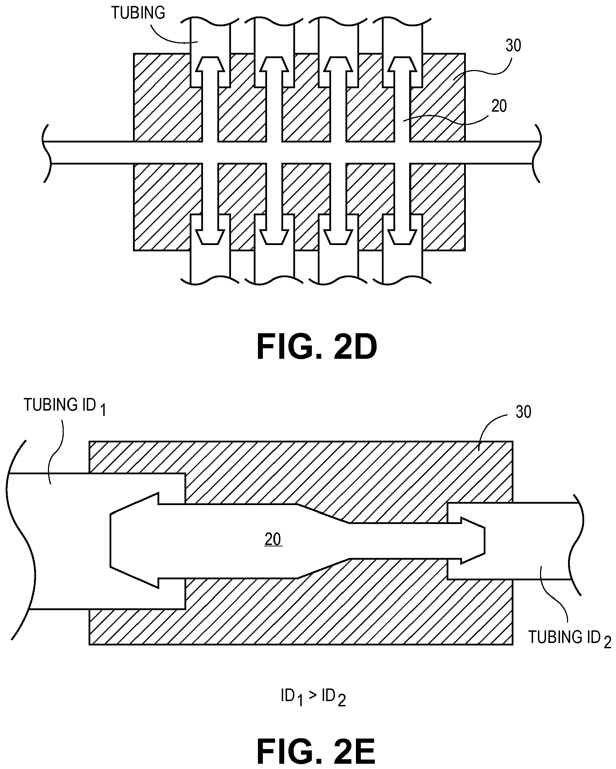

FIGS. 2A-2E illustrates various different embodiments of a fluid management assembly 10. The connector 20 may take a number of different forms and configurations. For example, the connector 20 may be a wye, tee, elbow, coupling, or connector. FIG. 2A illustrates a connector 20 in the form of a wye (or "Y"). FIG. 2B illustrates the connector 20 in the form of a coupler. FIG. 2C illustrates a connector in the form of an elbow. FIG. 2D illustrates another embodiment of a connector 20 in the form of a manifold. FIG. 2E illustrates another embodiment of a connector 20 in the form of a reducer. Here, the connector 20 is able to couple tubes having different internal diameters (ID). Other configurations may also be used for the connector 20. For example, the connector 20 may be a union member, or multi inlet/outlet member. The connector 20, in a general sense, includes at least one inlet and at least one outlet.

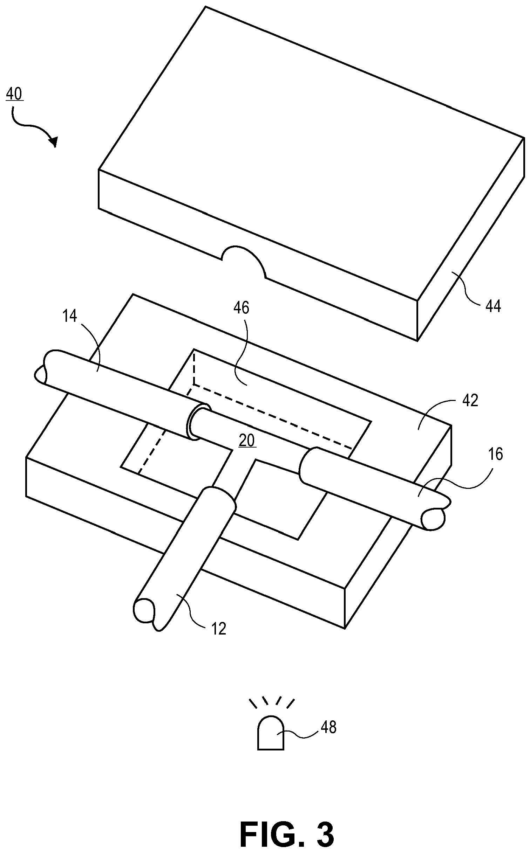

FIG. 3 illustrates a mold 40 used in connection with the formation of the fluid management assembly 10. The mold 40 illustrated in FIG. 3 includes first and second halves 42, 44. A cavity 46 is formed in the mold as illustrated in FIG. 3 that is used to receive the polymer-based overmolding 30 in liquid form. The mold 40 should be transparent to UV radiation and stable during the curing process. In one aspect, the mold 40 is made from quartz glass or polymethyl methacrylate (PMMA). A UV light source 48 is also provided as seen in FIG. 3. The UV light source 48 may be provided external to the mold 40 or, in other embodiments, may be integrated into a portion of the mold 40 itself. The UV light source 48 may include gas discharge lamps (microwave or electrode systems) or LED lamps. LED lamps may be preferred because they generate less heat and may result in molds 40 having a longer life. The molds 40 may be made using conventional mold-making processes. The molds 40 can also be made using three-dimensional printing technologies such that designs can be rapidly and cheaply turned into products.

In one aspect of the invention, a method of forming a fluid management assembly 10 includes coupling first and second silicone tubing portions (e.g., 12, 14, 16) to a connector 20 having at least one inlet and at least one outlet and a lumen extending there between, wherein the at least one inlet is coupled to the first silicone tubing portion at a first interface and wherein the at least one outlet is coupled to the second silicone tubing portion at a second interface. The connector 20 is inserted into a cavity of a mold 40. Alternatively, the silicone tubing portions 12, 14, 16 may be connected to the connecter 20 after it has been inserted into the mold 40. A liquid silicone rubber is injected into the mold cavity 46 and surrounds the connector 20 and connected tubing portions 12, 14, 16. UV radiation from the UV light source 48 is applied to the liquid silicone rubber to cure the silicone. While this embodiment is described in the context of a UV-curable silicone, it should be understood that other UV-curable polymers are also contemplated.

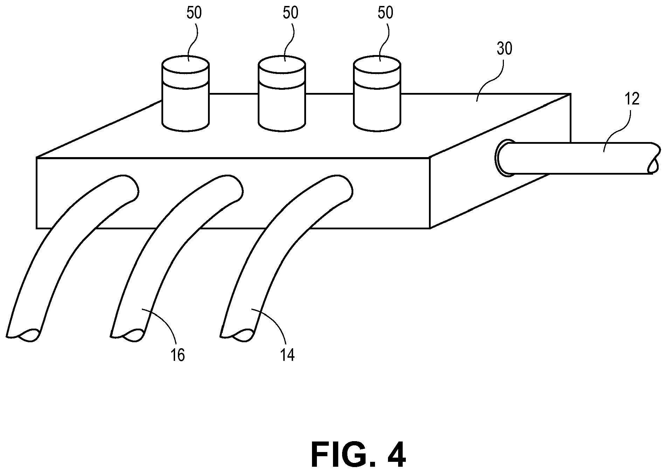

FIG. 4 illustrates another embodiment of a fluid management assembly 10. In this embodiment, the overmolding 30 encapsulates an interior connector like the manifold of FIG. 2D that is connected to varying tubing segments. In this embodiment, valves 50 are provided on the overmolding 30 and selectively engage or pinch portions of the interior connector to valve fluid to one or more particular tubing segments. The valves 50 may be manual bonnet-style valves or the valves may be automatically actuated valves (e.g., pneumatic or servo-based). The fluid management assembly 10 of FIG. 10 has particular robustness and can operate at high pressures.

It should be understood that the fluid management assembly 10 is designed to be disposable. Systems that are disposable or incorporate disposable elements are advantageous because they avoid the need for cleaning-in-place (CIP) cleaning, sanitization, or re-sterilization. A major advantage of the embodiments described herein is that the product contained within the system only contacts two components - the interior surface of the tubing 12, 14, 16 as well as the interior surface of the connector 20. The product does not contact any third substance such as a mandrel or flashing material left from some other component used during the manufacturing process. The fluid management assembly 10 is also scalable meaning that large diameter tubes can be used. Further, the fluid management assembly 10 is very robust and able to handle large pressures.

In another alternative embodiment of the invention, a fluid management assembly may be made using a mandrel using one or more techniques described above, for example, as noted in U.S. Patent 6,290,265, however the mandrel is used in conjunction with a UV-curable overmolding as described herein. In such an alternative embodiment, there is no need for a separate connector.

While embodiments of the present invention have been shown and described, various modifications may be made without departing from the scope of the present invention. The invention, therefore, should not be limited, except to the following claims.

## Claims

1. A fluid management assembly (10) comprising:
a connector (20) having at least one inlet and at least one outlet and a lumen extending there between, wherein the connector (20) comprises a plastic material comprising one of polyethylene (PE), polypropylene (PP), polyvinylidene fluoride (PVDF), perfluoroalkoxy (PFA), polytetrafluoroethylene (PTFE), or
polycarbonate;
a first silicone tubing (12) connected to the at least one inlet at a first interface; a second silicone tubing (14, 16) connected to the at least one outlet at a second interface;
a silicone overmolding (30) surrounding the connector (20), the first interface, and the second interface, wherein the silicone overmolding (30) comprises a UV-cured silicone cured at ambient or slightly above temperatures.

2. The fluid management assembly (10) of claim 1, wherein the connector (20) comprises a Tee connector, a Wye conector, a union, a coupler, an elbow, a reducer.

3. The fluid management assembly (10) of claim 1, further comprising a clamp (28) surrounding the first silicone tubing at the first interface.

4. The fluid management assembly (10) of claim 1, further comprising a clamp (28) surrounding the second silicone tubing at the second interface.

5. The fluid management assembly (10) of claim 1, wherein the burst pressure of the assembly exceeds 3.45 bar (50 psi).

6. The fluid management assembly (10) of claim 1, wherein the burst pressure of the assembly exceeds 6.89 bar (100 psi).

7. The fluid management assembly (10) of claim 1, wherein the at least one inlet and the at least one outlet comprise barbed ends (22, 24, 26) formed about the exterior of the connector.

8. The fluid management assembly of claim 1, further comprising valves disposed on the silicon overmolding and configured to selectively engage or pinch portions of the connector.

9. A method of forming a fluid management assembly (10) comprising:
coupling first and second silicone tubing portions to a plastic connector comprising one of polyethylene (PE), polypropylene (PP), polyvinylidene fluoride (PVDF), perfluoroalkoxy (PFA), polytetrafluoroethylene (PTFE), or
polycarbonate and having at least one inlet and at least one outlet and a lumen extending there between, wherein the at least one inlet is coupled to the first silicone tubing portion at a first interface and wherein the at least one outlet is coupled to the second silicone tubing portion at a second interface;
inserting the connector (20) into a cavity of a mold (40);
injecting a liquid silicone rubber into the mold cavity cured at ambient or slightly above temperatures; and
applying UV radiation to the liquid silicone rubber through the mold to cure the silicone.

10. The method of claim 9, wherein the connector (20) is inserted into the cavity of the mold (40) prior to coupling the first and second silicone tubing portions to the plastic material connector (20).

11. The method of claim 9, wherein the connector (20) is inserted into the cavity of the mold (40) after coupling the first and second silicone tubing portions to the plastic material connector (20).

12. The method of claim9, further comprising securing a clamp (28) around the first silicone tubing portion at the first interface or a clamp around the second silicone tubing portion at the second interface.

## Patentansprüche

1. Fluidmanagementanordnung (10), umfassend:
einen Verbinder (20), der mindestens einen Einlass und mindestens einen Auslass und ein sich dazwischen erstreckendes Lumen aufweist, wobei der Verbinder (20) ein Kunststoffmaterial umfasst, das Polyethylen (PE), Polypropylen (PP),
Polyvinylidenfluorid (PVDF), Perfluoralkoxy (PFA), Polytetrafluorethylen (PTFE) oder Polycarbonat umfasst;
einen ersten Silikonschlauch (12), der mit dem mindestens einen Einlass an einer ersten Verbindungsstelle verbunden ist; einen zweiten Silikonschlauch (14, 16), der mit dem mindestens einen Auslass an einer zweiten Verbindungsstelle verbunden ist;
eine Silikonüberspritzung (30), die den Verbinder (20), die erste Verbindungsstelle und die zweite Verbindungsstelle umgibt, wobei die Silikonüberspritzung (30) ein UV-gehärtetes Silikon umfasst, das bei Umgebungstemperatur oder geringfügig höheren Temperaturen gehärtet wurde.

2. Fluidmanagementvorrichtung (10) nach Anspruch 1, wobei der Verbinder (20) einen T-Verbinder, einen Y-Verbinder, eine Verschraubung, eine Kupplung, einen Rohrbogen, ein Reduzierstück umfasst.

3. Fluidmanagementanordnung (10) nach Anspruch 1, ferner umfassend eine Klemme (28), die den ersten Silikonschlauch an der ersten Verbindungsstelle umgibt.

4. Fluidmanagementanordnung (10) nach Anspruch 1, ferner umfassend eine Klemme (28), die den zweiten Silikonschlauch an der zweiten Verbindungsstelle umgibt.

5. Fluidmanagementanordnung (10) nach Anspruch 1, wobei der Berstdruck der Anordnung 3,45 bar (50 psi) überschreitet.

6. Fluidmanagementanordnung (10) nach Anspruch 1, wobei der Berstdruck der Anordnung 6,89 bar (100 psi) überschreitet.

7. Fluidmanagementanordnung (10) nach Anspruch 1, wobei der mindestens eine Einlass und der mindestens eine Auslass Widerhakenenden (22, 24, 26) umfassen, die um die Außenseite des Verbinders herum ausgebildet sind.

8. Fluidmanagementanordnung nach Anspruch 1, ferner umfassend Ventile, die auf der Silikonüberspritzung angeordnet und konfiguriert sind, um selektiv mit Abschnitten des Verbinders in Eingriff zu kommen oder diese einzuklemmen.

9. Verfahren zum Ausbilden einer Fluidmanagementanordnung (10), umfassend:
Koppeln erster und eines zweiter Silikonschlauchabschnitte mit einem Kunststoffverbinder, der Polyethylen (PE), Polypropylen (PP), Polyvinylidenfluorid (PVDF), Perfluoralkoxy (PFA), Polytetrafluorethylen (PTFE) oder Polycarbonat umfasst und mindestens einen Einlass und mindestens einen Auslass und ein sich dazwischen erstreckendes Lumen aufweist, wobei der mindestens eine Einlass mit dem ersten Silikonschlauchabschnitt an einer ersten Verbindungsstelle gekoppelt ist und wobei der mindestens eine Auslass mit dem zweiten Silikonschlauchabschnitt an einer zweiten Verbindungsstelle gekoppelt ist; Einsetzen des Verbinders (20) in einen Hohlraum einer Form (40);
Einspritzen eines flüssigen Silikonkautschuks in den Formhohlraum, der bei Umgebungstemperatur oder geringfügig höheren Temperaturen gehärtet wird; und
Anwenden von UV-Strahlung auf den flüssigen Silikonkautschuk durch die Form, um das Silikon zu härten.

10. Verfahren nach Anspruch 9, wobei der Verbinder (20) in den Hohlraum der Form (40) eingesetzt wird, bevor die ersten und zweiten Silikonschlauchabschnitte mit dem Kunststoffverbinder (20) gekoppelt werden.

11. Verfahren nach Anspruch 9, wobei der Verbinder (20) in den Hohlraum der Form (40) eingesetzt wird, nachdem die ersten und zweiten Silikonschlauchabschnitte mit dem Kunststoffverbinder (20) gekoppelt wurden.

12. Verfahren nach Anspruch 9, ferner umfassend das Sichern einer Klemme (28) um den ersten Silikonschlauchabschnitt an der ersten Verbindungsstelle oder einer Klemme um den zweiten Silikonschlauchabschnitt an der zweiten Verbindungsstelle.

## Revendications

1. Assemblage de gestion de fluide (10) comprenant :
un raccord (20) possédant au moins une entrée et au moins une sortie et une lumière s'étendant entre les deux ; dans lequel le raccord (20) comprend une matière plastique comprenant une matière choisie parmi du polyéthylène (PE), du polypropylène (PP), du fluorure de polyvinylidène (PVDF), du perfluoroalcoxy (PFA), du polytétrafluoréthylène (PTFE) ou du polycarbonate ;
une première tubulure en silicone (12) reliée à ladite au moins une entrée à une première interface ;
une seconde tubulure en silicone (14, 16) reliée à ladite au moins une sortie à une seconde interface ;
un surmoulage en silicone (30) entourant le raccord (20), la première interface et la seconde interface, dans lequel le surmoulage en silicone (30) comprend une silicone durcie par exposition au rayonnement ultraviolet, durcie à la température ambiante ou à des températures légèrement supérieures.

2. Assemblage de gestion de fluide (10) selon la revendication 1, dans lequel le raccord (20) comprend un raccord en T, un raccord en Y, un raccord union, un coupleur, un coude, un réducteur.

3. Assemblage de gestion de fluide (10) selon la revendication 1, comprenant en outre un collier (28) entourant la première tubulure en silicone à la première interface.

4. Assemblage de gestion de fluide (10) selon la revendication 1, comprenant en outre un collier (28) entourant la seconde tubulure en silicone à la seconde interface.

5. Assemblage de gestion de fluide (10) selon la revendication 1, dans lequel la pression de rupture de l'assemblage est supérieure à 3,45 bar (50 psi).

6. Assemblage de gestion de fluide (10) selon la revendication 1, dans lequel la pression de rupture de l'assemblage est supérieure à 6,89 bar (100 psi).

7. Assemblage de gestion de fluide (10) selon la revendication 1, dans lequel ladite au moins une entrée et ladite au moins une sortie comprennent des extrémités barbelées (22, 24, 26) réalisées sur la face externe du raccord.

8. Assemblage de gestion de fluide (10) selon la revendication 1, comprenant en outre des clapets disposés sur le surmoulage en silicone et configurés pour, de manière sélective, entrer en contact ou pincer des portions du raccord.

9. Procédé de formation d'un assemblage de gestion de fluide (10) comprenant le fait de :
coupler une première portion et une seconde portion de tubulure en silicone à un raccord en matière plastique comprenant une matière choisie parmi du polyéthylène (PE), du polypropylène (PP), du fluorure de polyvinylidène (PVDF), du perfluoroalcoxy (PFA), du polytétrafluoréthylène (PTFE) ou du polycarbonate et possédant au moins une entrée et au moins une sortie et une lumière s'étendant entre les deux ; dans lequel ladite au moins une entrée est couplée à la première portion de tubulure en silicone à une première interface et dans lequel ladite au moins une sortie est couplée à la seconde portion de tubulure en silicone à une seconde interface ;
insérer le raccord (20) dans une cavité d'un moule (40) ;
injecter un caoutchouc de silicone liquide dans la cavité de moule, durci à la température ambiante ou à des températures légèrement supérieures ; et
appliquer un rayonnement ultraviolet au caoutchouc de silicone liquide à travers le moule dans le but de durcir la silicone.

10. Procédé selon la revendication 9, dans lequel le raccord (20) est inséré dans la cavité du moule (40) avant le couplage de la première portion et de la seconde portion de tubulure en silicone au raccord en matière plastique (20).

11. Procédé selon la revendication 9, dans lequel le raccord (20) est inséré dans la cavité du moule (40) après le couplage de la première portion et de la seconde portion de tubulure en silicone au raccord en matière plastique (20).

12. Procédé selon la revendication 9, comprenant en outre le fait de fixer un collier (28) autour de la première portion de tubulure en silicone à la première interface ou un collier autour de la seconde portion de tubulure en silicone à la seconde interface.
